# EUROPEAN PATENT SPECIFICATION

(11) **EP 3 836 906 B1**
(45) Date of publication and mention of the grant of the patent: **29.03.2023**
(21) Application number: 19759058.1
(22) Date of filing: 13.08.2019
(51) Int. Cl.: A61K 9/46, A61K 9/00, A61K 9/20, A61K 9/28

(54) **EXPANDABLE DRUG DELIVERY PILL**
EXPANDIERBARE ARZNEIMITTELABGABEPILLE
PILULE EXTENSIBLE POUR L'ADMINISTRATION DE MÉDICAMENTS

(30) Priority: 14.08.2018 US 201816103420
(43) Date of publication of application: 23.06.2021
(73) Proprietor: Alma Therapeutics Ltd., 6228523 Petach Tilkva (IL)
(72) Inventor: GROSS, Yossi, 73160 Moshav Mazor (IL); CABIRI, Oz, 4526611 Hod Hasharon (IL); BEN-SHITRIT, Lital, 7822806 Ashkelon (IL)
(74) Representative: Marks & Clerk LLP
(86) International application number: PCT/IL2019/050906
(87) International publication number: WO 2020/035857

(56) References cited:
- WO-A1-2014/159604
- WO-A1-2015/120471
- US-A1- 2016 015 648

## Description

### CROSS-REFERENCE TO RELATED APPLICATIONS

The present application claims priority from and is a continuation-in-part of US Application 16/103,420, filed August 14, 2018.

### FIELD OF THE INVENTION

The present invention generally relates to medical apparatus. Specifically, the present invention relates to an ingestible capsule for administering medication to a subject.

### BACKGROUND OF THE INVENTION

Medication is frequently stored in a capsule and administered to a subject who swallows the capsule. The medication passes through the intestinal wall and enters the blood of the subject.

US Patent 8,287,902 to Gross describes an ingestible capsule for delivering medication to a subject. A capsule coating dissolves in a gastrointestinal tract of the subject. An inner core of the capsule has an outer surface associated therewith. The outer surface is disposed within the coating and expands when the coating dissolves. A medication is disposed on the outer surface, and the outer surface is configured such that the medication contacts an intestinal wall of the subject when the outer surface expands.

US Patent 9,492,396 to Gross describes an ingestible pill includes a coating configured to dissolve in a small intestine; a core, which includes a medication-delivery element, which (a) has a compressed shape when disposed within the coating, and (b) is configured to assume, after the coating dissolves, an expanded shape; a medication; and a mucoadhesive. When unconstrained in the expanded shape, the medication-delivery element (a) is shaped so as to define first and second surfaces on opposite sides of the medication-delivery element, which have respective outer perimeters, which surround respective spaces of the respective surfaces, which spaces have respective greatest dimensions equal to between 2 and 10 cm, and each of which spaces has an area equal to at least 50% of the square of the greatest dimension; and (b) has an average thickness between the first and the second surfaces of less than 6mm. Each of the medication and the mucoadhesive at least partially coats the first surface.

US-A-2016-015648 discloses an ingestible pill including a coating configured to dissolve in a small intestine; a core, which includes a medication-delivery element, which (a) has a compressed shape when disposed within the coating, and (b) is configured to assume, after the coating dissolves, an expanded shape; a medication; and a mucoadhesive.

WO-A-2015-120471 discloses devices including multilayered bioadhesive patches delivered orally by dissolvable ingestible capsule, wherein various mechanisms are provided for anchoring the bioadhesive patches to the duodenal mucosa.

WO-A-2014-159604 discloses embodiments providing swallowable devices, preparations and methods for delivering drugs and other therapeutic agents within the GI tract. Particular embodiments provide a swallowable device such as a capsule for delivering drugs into the intestinal wall or other GI lumen.

### SUMMARY OF THE APPLICATION

The invention is set out in the appended set of claims.

### BRIEF DESCRIPTION OF THE DRAWINGS

Figs. 1A-B are schematic illustrations of an ingestible pill, for ingestion by a subject.
Figs. 2A-F are schematic illustrations of a medication-delivery device of an ingestible pill.
Figs. 3A-B are schematic illustrations of a medication-delivery device of another ingestible pill.
Figs. 4A-F are schematic illustrations of a medication-delivery device of yet another ingestible pill.
Figs. 5A-B are schematic illustrations of a medication-delivery device of still another ingestible pill.
Figs. 6A-F are schematic illustrations of a medication-delivery device of another ingestible pill.
Figs. 7A-C are schematic illustrations of a medication-delivery device of an ingestible pill.
Figs. 8A-B are schematic illustration of a medication-delivery device of an ingestible pill.
Figs. 9A-B are schematic illustrations of a medication-delivery device of an ingestible pill.
Figs. 10A-B are schematic illustrations of a medication-delivery device of an ingestible pill.
Figs. 11A-B are schematic illustrations of a medication-delivery device of an ingestible pill.
Figs. 12A-C which are schematic illustrations of configurations of a patch.
Figs. 13A-D are schematic illustrations of configurations of another patch.
Fig. 14 is a schematic illustration of another ingestible pill, for ingestion by a subject, in accordance with an application of the present invention;
Fig. 15 is a schematic illustration of a medication-delivery device of yet another ingestible pill, for ingestion by a subject, in accordance with an application of the present invention;
Fig. 16 is a schematic illustration of a method of rolling a patch of the medication-delivery devices of Figs. 14 and 15, in accordance with an application of the present invention;
Figs. 17A-C are schematic illustrations of three-panel folds known in the art of paper folding; and
Figs. 18A-D are schematic illustrations of several configurations of a patch of the ingestible pill of Fig. 14, in accordance with respective applications of the present invention.

### DETAILED DESCRIPTION OF EMBODIMENTS

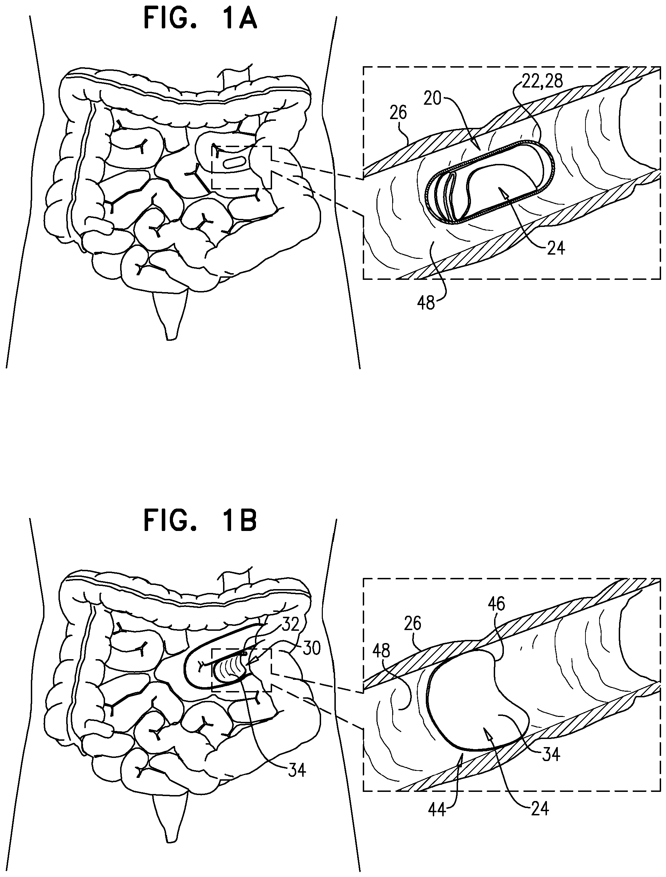

Figs. 1A-B are schematic illustrations of an ingestible pill 20, for ingestion by a subject.

Ingestible pill 20 comprises an enteric coating 22 and a medication-delivery device 24.

Enteric coating 22 is configured to dissolve in a small intestine 26 (e.g., a duodenum, jejunum, and/or ileum) of the subject. Typically, enteric coating 22 is pH-sensitive, and may be configured to dissolve within 10 minutes (e.g., within 5 minutes) within a range of pH values, which range has a low end of between 6.5 and 8.5 (and, optionally, a high end of between 7.5 and 14, such as between 7.5 and 9.5). For example, enteric coating 22 may comprise gelatin or starch. For some applications, as shown in Fig. 1A, pill 20 comprises a shell 28, which comprises enteric coating 22. Alternatively, for some applications, medication-delivery device 24 is directly coated with enteric coating 22 (configuration not shown). Typically, pill 20 has a length of at least 5 mm, no more than 30 mm, and/or between 5 and 30 mm before the enteric coating dissolves.

Medication-delivery device 24 comprises (a) a patch 30 having an upper (intestinal-wall-contact) surface 32 and a lower (intestinal-lumen-facing) surface 34, which face in generally opposite directions, and (b) needles 40. Medication-delivery device 24 (a) has a compressed shape 42 when disposed within coating 22, as shown in Fig. 1A, and (b) is configured to assume, after enteric coating 22 dissolves, an expanded shape 44, in which patch 30 has an outer perimeter 46. Fig. 1B shows medication-delivery device 24 in expanded shape 44, constrained by a wall 48 of small intestine 26. Typically, patch 30 comprises a non-metal material, such as an elastic material (e.g., polyurethane or silicone), or alternatively a shape memory material such as Nitinol, which, for example, may be set to change into a super-elastic condition after reaching a temperature of 32-34 degrees C. Medication-delivery device 24 expands, such as by stretching, unfolding, and/or unrolling, in response to no longer being constrained by enteric coating 22, and/or in response to contact of medication-delivery device 24 with fluid in small intestine 26.

Typically, each of needles 40 has a length of at least 20 microns (e.g., at least 50 microns), no more than 600 microns (e.g., no more than 300 microns, such as no more than 250 microns), and/or between 20 and 600 microns, e.g., between 20 and 600 microns, e.g., between 20 and 300 microns, such as between 50 and 250 microns. Typically, a greatest dimension of needles 40, measured perpendicular to a long axis, is between 40 and 300 microns. For some applications, needles 40 are conical or pyramidal, and the greatest dimension is thus at the bases of the cones or pyramids of the needles.

For some applications, medication-delivery device 24 is biodegradable in small intestine 26. For some applications, patch 30 comprises an elastomer, such as polyethylene or silicone.

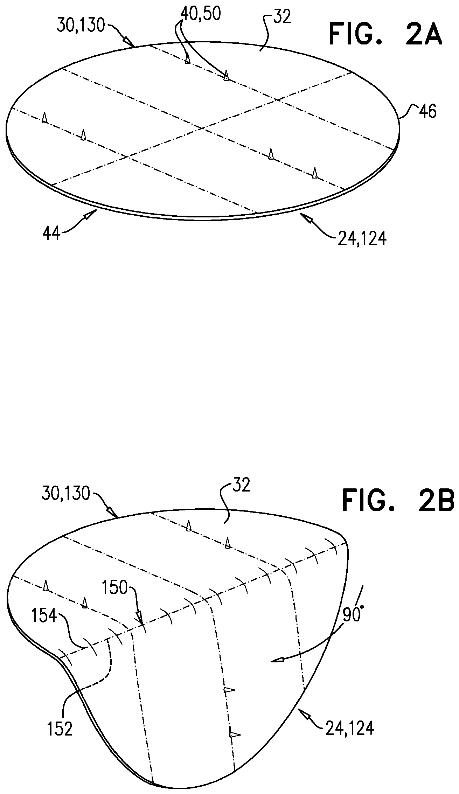

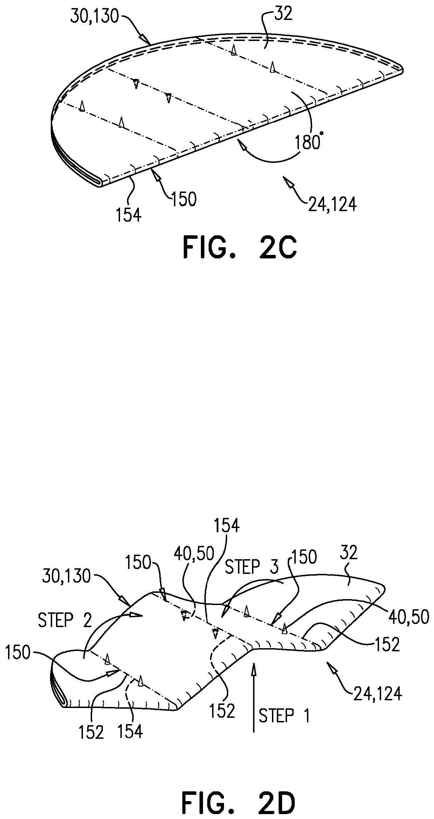

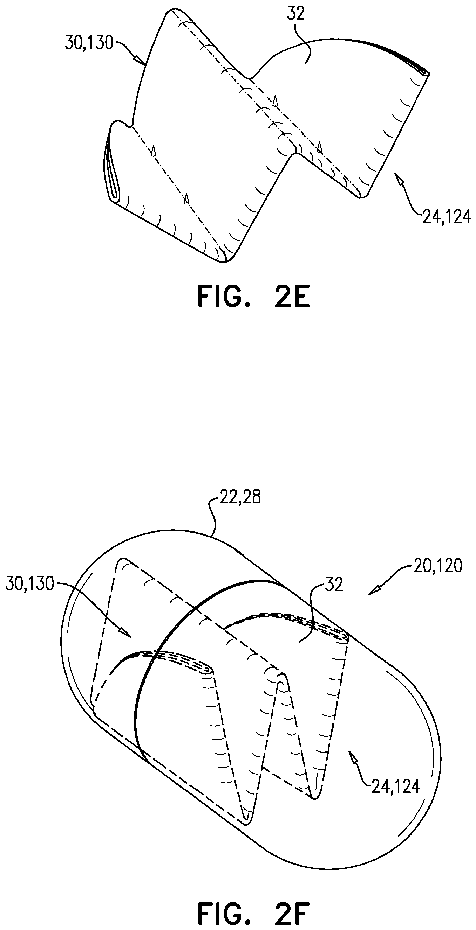

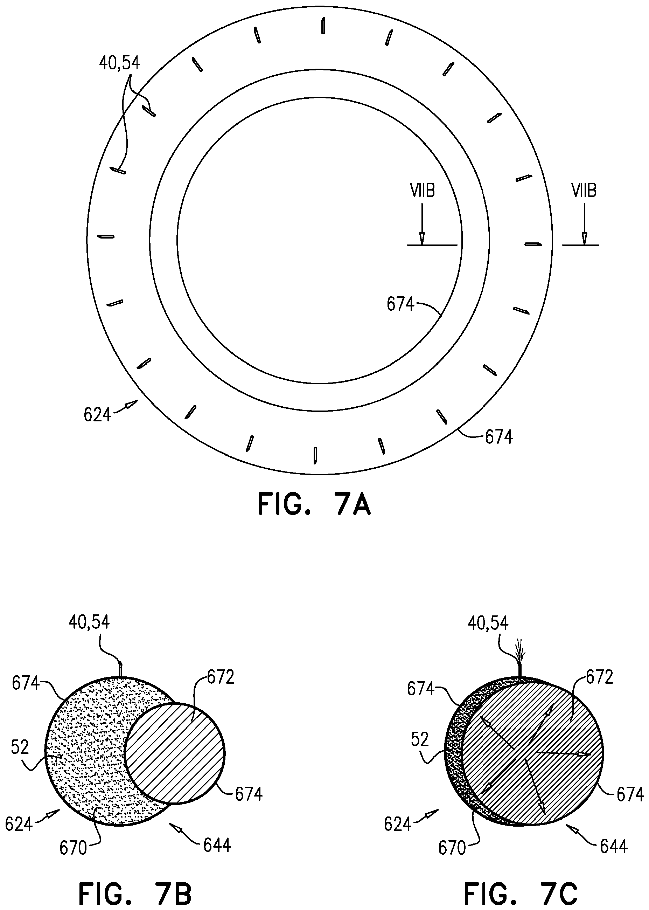

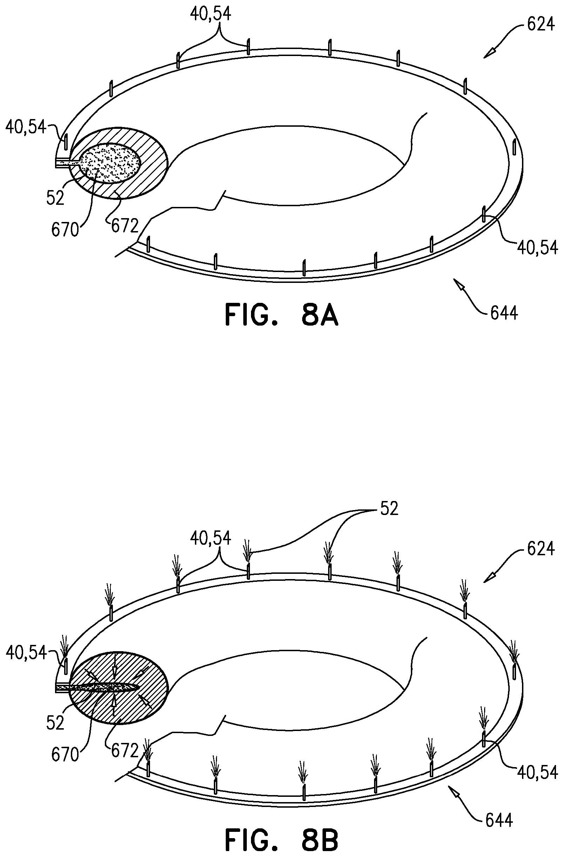

For some applications, such as shown in Figs. 1A-B and described hereinbelow with reference to Figs. 2A-F, 3A-B, 4A-F, 5A-B, and 6A-F, needles 40 are medication-needles 50, which comprise a solid medication (for example, the solid medication may be integrated in a glucose structure). For other applications, such as described hereinbelow with reference to Figs. 7A-C, 8A-B, 9A-B, and 10A-B, medication-delivery device 24 further comprises a medication 52 (e.g., a liquid medication), and needles 40 are medication-delivery needles 54, which are configured to deliver medication 52. For these applications, medication-delivery needles 54 are typically hollow and are configured to deliver medication 52.

Typically, patch 30 is configured such that expanded shape 44 is generally flat when medication-delivery device 24 is unconstrained, such as shown in Figs. 2A, 3A, 4A, 5A, and 6A, and assumes a curved shape when constrained by wall 48 of small intestine 26, such as shown in Fig. 1B. Ingestible pill 20 is configured such that when patch 30 assumes expanded shape 44 upon dissolving of enteric coating 22 in small intestine 26, upper surface 32 of patch 30 contacts intestinal wall 48, thereby bringing needles 40 into contact with intestinal wall 48. Typically, but not necessarily, needles 40 penetrate intestinal wall 48, typically the villi thereof, in order to enhance uptake of the medication. In addition, needles 40 may also serve as temporary anchors that hold patch 30 in place during medication delivery. Medication-delivery device 24 typically remains axially stationary in the small intestine long enough for needles 40 to slowly push into tissue of intestinal wall 48, such as because of peristalsis, and to inject medication, for applications in which the needles are hollow, or to dissolve, for applications in which the needles comprise medication. For some applications in which medication-delivery device 24 expands (e.g., inflates), the expansion itself does not generally push the needles into the intestinal wall. Following delivery of the medication, medication-delivery device 24 is passed from the body.

Once expanded, upper surface 32 of patch 30 establishes good (complete or nearly complete) contact with intestinal wall 48, as shown in Fig. 1B. The shape and dimensions of medication-delivery device 24 may contribute to this good contact by preventing other, lower-contact-level orientations of the patch in the lumen of small intestine 26. The location of patch 30 is generally unaffected by peristalsis, and does not block food passing through the intestine.

For some applications, medication-delivery device 24 further comprises a mucoadhesive that at least partially coats upper surface 32. For example, the mucoadhesive may be sprayed or printed on upper surface 32 using techniques known in the art. The mucoadhesive transiently helps adhere upper surface 32 in position against intestinal wall 48 during delivery of the medication through intestinal wall 48. For example, the mucoadhesive may include an adhesive agent described in US Patent 6,235,313 to Mathiowitz et al., or in an article by Tao et al., Tao et al., entitled, "Gastrointestinal patch systems for oral drug delivery," Drug Discovery Today, Vol. 10(13), July 2005.

Reference is made to Figs. 2A-F, which are schematic illustrations of a medication-delivery device 124 of an ingestible pill 120.

Fig. 2A shows medication-delivery device 124 when unconstrained in expanded shape 44, Figs. 2B-E show medication-delivery device 124 in various stages of folding, and Fig. 2F shows medication-delivery device 124 folded within enteric coating 22. Medication-delivery device 124 is one configuration of medication-delivery device 24, described hereinabove with reference to Figs. 1A-B, and may implement any of the features described with reference to Figs. 1A-B. Medication-delivery device 124 comprises a patch 130, which is one configuration of patch 30, described hereinabove with reference to Figs. 1A-B, and may implement any of the features described with reference to Figs. 1A-B. In general, the folding pattern and disposition of patch 130 within enteric coating 22 determines which surface of the patch comes in contact with intestinal wall 48 upon dissolving of enteric coating 22.

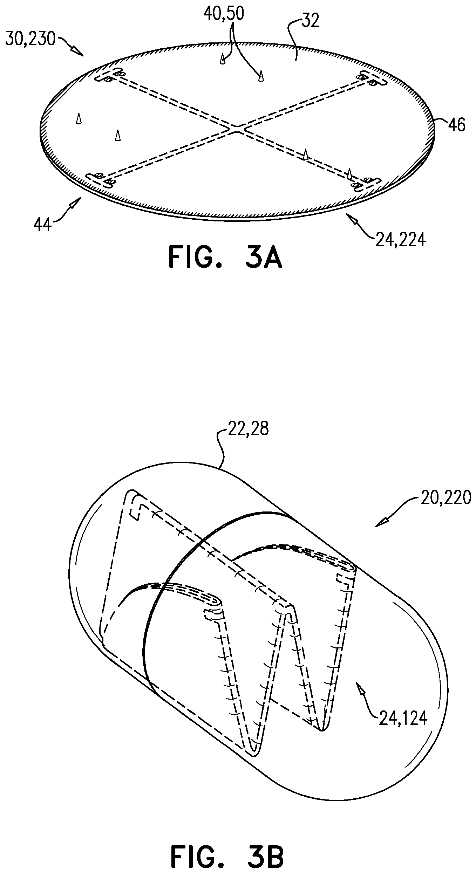

Reference is made to Figs. 3A-B, which are schematic illustrations of a medication-delivery device 224 of an ingestible pill 220.

Fig. 3A shows medication-delivery device 224 when unconstrained in expanded shape 44, and Fig. 3B shows medication-delivery device 224 folded within enteric coating 22. Medication-delivery device 224 is one configuration of medication-delivery device 24, described hereinabove with reference to Figs. 1A-B, and may implement any of the features described with reference to Figs. 1A-B. Other than as described below, medication-delivery device 224 is similar to medication-delivery device 124, and may implement any of the features thereof. Medication-delivery device 224 comprises a patch 230, which is one configuration of patch 30, described hereinabove with reference to Figs. 1A-B, and may implement any of the features described with reference to Figs. 1A-B.

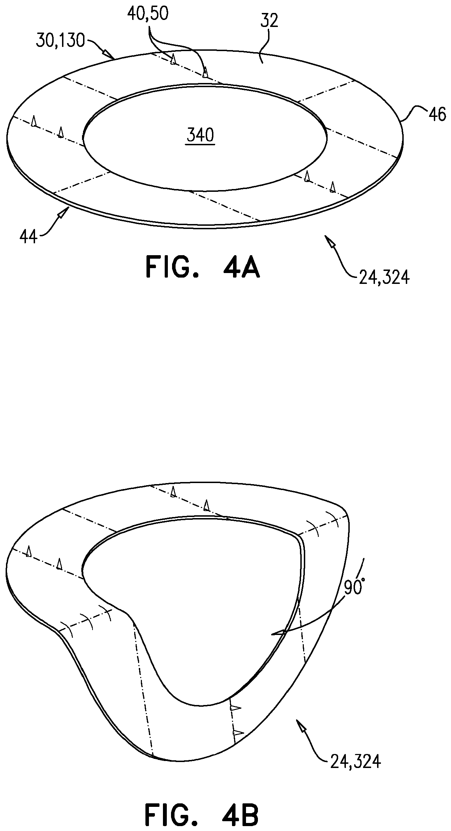

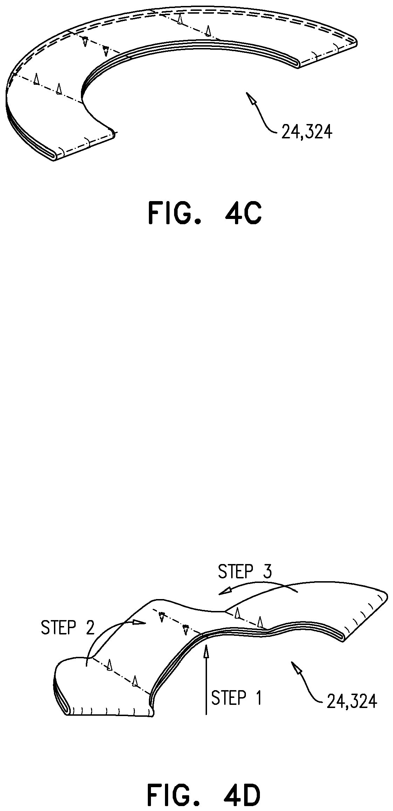

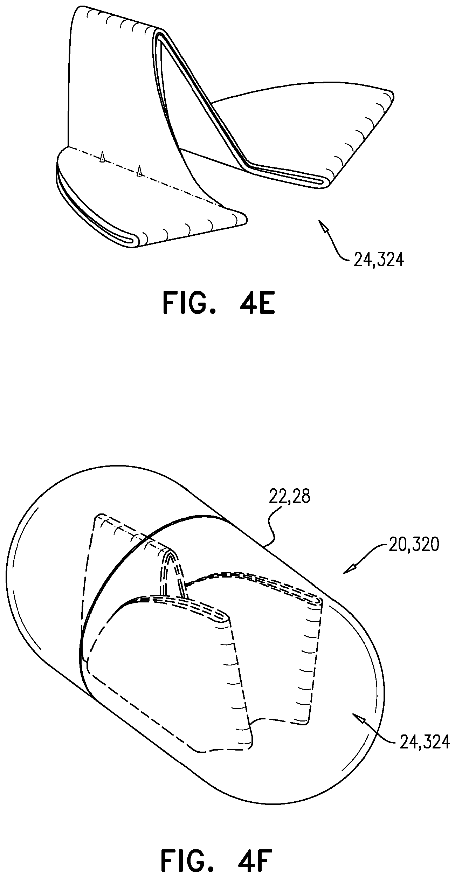

Reference is made to Figs. 4A-F, which are schematic illustrations of a medication-delivery device 324 of an ingestible pill 320.

Fig. 4A shows medication-delivery device 324 when unconstrained in expanded shape 44, Figs. 4B-E show medication-delivery device 124 in various stages of folding, and Fig. 4F shows medication-delivery device 324 folded within enteric coating 22. Medication-delivery device 324 is one configuration of medication-delivery device 24, described hereinabove with reference to Figs. 1A-B, and may implement any of the features described with reference to Figs. 1A-B. Other than as described below, medication-delivery device 324 is similar to medication-delivery device 124, and may implement any of the features thereof. Medication-delivery device 324 comprises a patch 330, which is one configuration of patch 30, described hereinabove with reference to Figs. 1A-B, and may implement any of the features described with reference to Figs. 1A-B.

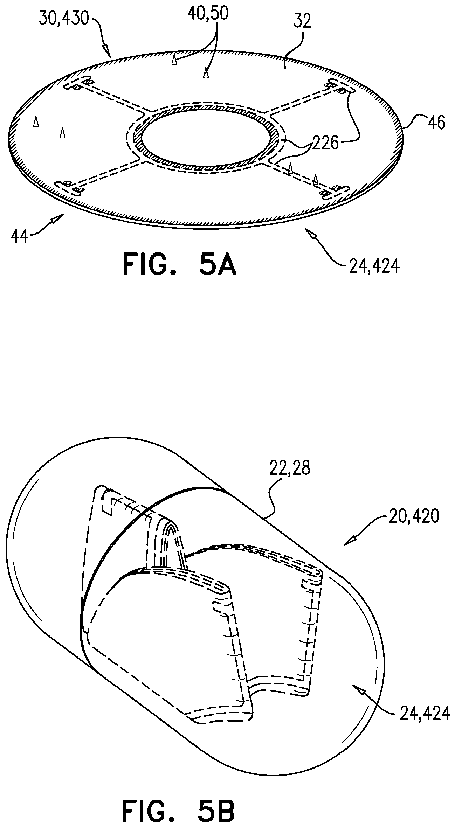

Reference is made to Figs. 5A-B, which are schematic illustrations of a medication-delivery device 424 of an ingestible pill 420.

Fig. 5A shows medication-delivery device 424 when unconstrained in expanded shape 44, and Fig. 5B shows medication-delivery device 424 folded within enteric coating 22. Medication-delivery device 424 is one configuration of medication-delivery device 24, described hereinabove with reference to Figs. 1A-B, and may implement any of the features described with reference to Figs. 1A-B. Other than as described below, medication-delivery device 424 is similar to medication-delivery device 324, and may implement any of the features thereof. Medication-delivery device 424 comprises a patch 130, which is one configuration of patch 430, described hereinabove with reference to Figs. 1A-B, and may implement any of the features described with reference to Figs. 1A-B.

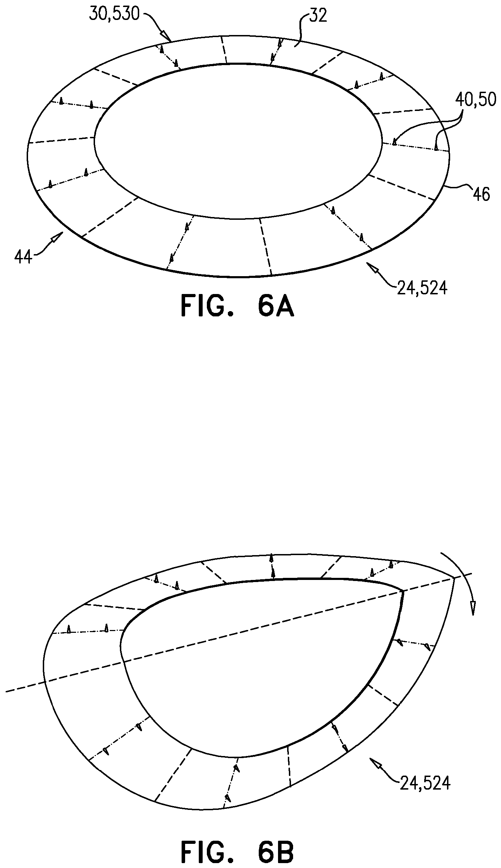

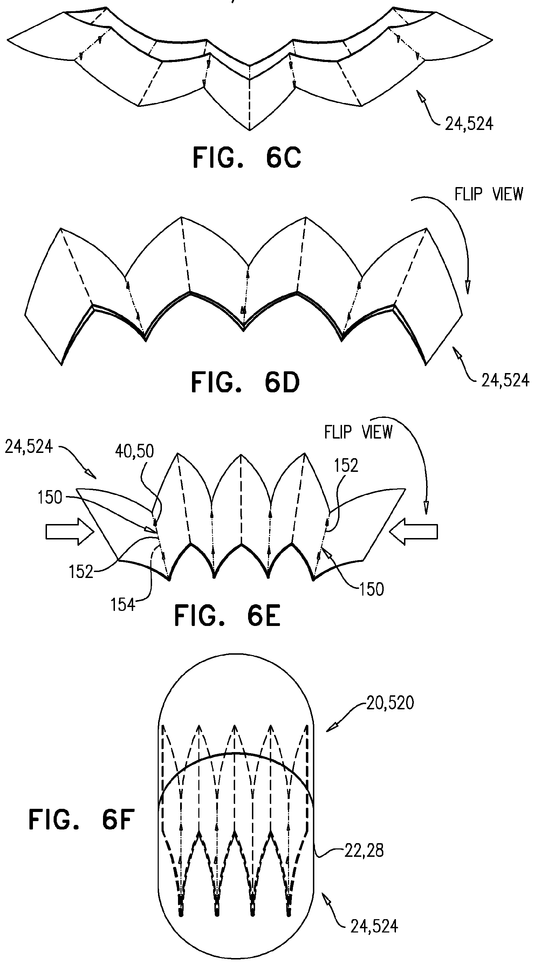

Reference is made to Figs. 6A-F, which are schematic illustrations of a medication-delivery device 524 of an ingestible pill 520.

Fig. 6A shows medication-delivery device 524 when unconstrained in expanded shape 44, Figs. 6B-E show medication-delivery device 124 in various stages of folding, and Fig. 6F shows medication-delivery device 524 folded within enteric coating 22. Medication-delivery device 524 is one configuration of medication-delivery device 24, described hereinabove with reference to Figs. 1A-B, and may implement any of the features described with reference to Figs. 1A-B. Other than as described below, medication-delivery device 524 is similar to medication-delivery device 324, and may implement any of the features thereof. Medication-delivery device 524 comprises a patch 530, which is one configuration of patch 30, described hereinabove with reference to Figs. 1A-B, and may implement any of the features described with reference to Figs. 1A-B.

Reference is made to Figs. 2A-F, 3A-B, 4A-F, 5A-B, and 6A-F. As shown in Figs. 2F, 3B, 4F, 5B, and 6F, patch 30 is disposed within enteric coating 22, folded so as to define one or more creases 150 (e.g., a plurality of creases 150), which define respective inner crease sides 152 and outer crease sides 154. The formation of these creases is illustrated in Figs. 2A-E, 3A, 4A-E, 5A, and 6A-E, respectively. As used in the present application, including in the claims, a "crease" is a sharp, well-defined fold. For some applications, at least 50% (such as at least 75%, e.g., at least 90%, e.g., 100%) of needles 40 are coupled (e.g., adhered) to patch 30 along inner crease sides 152. Coupling needles 40 to patch along inner crease sides 152 helps protect the needles from being broken during folding of the patch and stabilizes the needles on either side of the crease, and helps avoiding deflecting the needles from the plane of the surface when the patch is folded.

For some applications, patch 30 is disposed within enteric coating 22, folded first in half (as can perhaps best be seen in Figs. 2B-C, 4B-C, and 6B), and then accordion-folded, as can be seen in Figs. 2D-F, 3B, 4D-F, 5B, and 6C-F.

Typically, patch 30 is disposed within enteric coating 22, folded such that when patch 30 assumes expanded shape 44 upon dissolving of enteric coating 22 in small intestine 26, upper surface 32 of patch 30 contacts intestinal wall 48, thereby bringing needles 40 into contact with intestinal wall 48. In other words, the folding of patch 30 within enteric coating 22 typically determines which surface of patch 30 comes in contact with intestinal wall 48.

Typically, needles 40 are not coupled to respective inner crease sides 152 of a portion of creases 150. Also typically, needles 40 are not coupled to respective inner crease sides 152 of two or more of creases 150.

For some applications, at least 50%, such as at least 80%, e.g., 100%, of needles 40 are coupled to upper surface 32 of patch 30 when patch 30 is disposed within enteric coating 22. Alternatively or additionally, for some applications, at least 50% of needles 40 are coupled to upper surface 32 of patch 30 along inner crease sides 152 when patch 30 is disposed within enteric coating 22.

Typically, between 0% (i.e., none) and 10% of needles 40 are coupled to patch 30 along outer crease sides 154 when patch 30 is disposed within enteric coating 22.

Reference is made to Figs. 2A, 3A, 4A, 5A, and 6A, which show patch 30 generally flat in expanded shape 44 when medication-delivery device 24 is unconstrained. For some applications, patch 30 can inscribe a circle having a diameter of at least 2 cm, less than 10 cm (e.g., less than 7 cm, such as less than 5 cm), and/or between 2 and 10 cm, e.g., between 2 and 7 cm, such as between 2 and 5 cm), when patch 30 assumes expanded shape 44 and is unconstrained. This diameter may allow patch 30 to circumscribe at least 180 degrees of the intestinal lumen, but typically not more than 360 degrees. (As is known in the art, an inscribed circle is the largest possible circle that can be drawn on the inside of a plane figure. It is noted that the circle is a geometric shape used to describe patch 30, and is not an element of the invention.) Alternatively or additionally, for applications in which patch 30 is circular, the diameter of patch 30 is typically greater than π (pi) times the radius of small intestine 26, and/or greater than 60 mm or between 65 mm and 150 mm, such that patch 30 extends around at least 180 degrees of a circumference of small intestine 26 when expanded in small intestine 26.

Reference is made to Figs. 4A-F, 5A-B, 6A-F, and 13A-D. In the configurations shown in these figures, patch 30 is annular, i.e., is shaped as a ring defining an opening 340 therethrough when unconstrained in expanded shape 44, as shown in Figs. 4A, 5A, 6A, and 13A-D. Opening 340 may allow for compression of medication-delivery device 24 when in compressed shape 42 when disposed within enteric coating 22, while still providing good contact with intestinal wall 48 when in expanded shape 44.

Reference is made to Figs. 3A-B and 5A-B. In the configurations shown in these figures, patch 30 comprises a non-metal material, and medication-delivery device 224, 424 further comprises elastic struts 226, which are fixed to patch 30. Elastic struts 226 are configured, upon enteric coating 22 dissolving, to transition medication-delivery device 224, 424 from the compressed shape to expanded shape 44.

For some applications, elastic struts 226 comprise metal, such as a shape memory alloy, such as Nitinol. The low stress of the shape memory alloy when the medication-delivery device has compressed shape 42 when disposed within coating 22 reduces the likelihood of the medication-delivery device forgetting its memorized shape. Alternatively, for some applications, struts 226 comprise stainless steel.

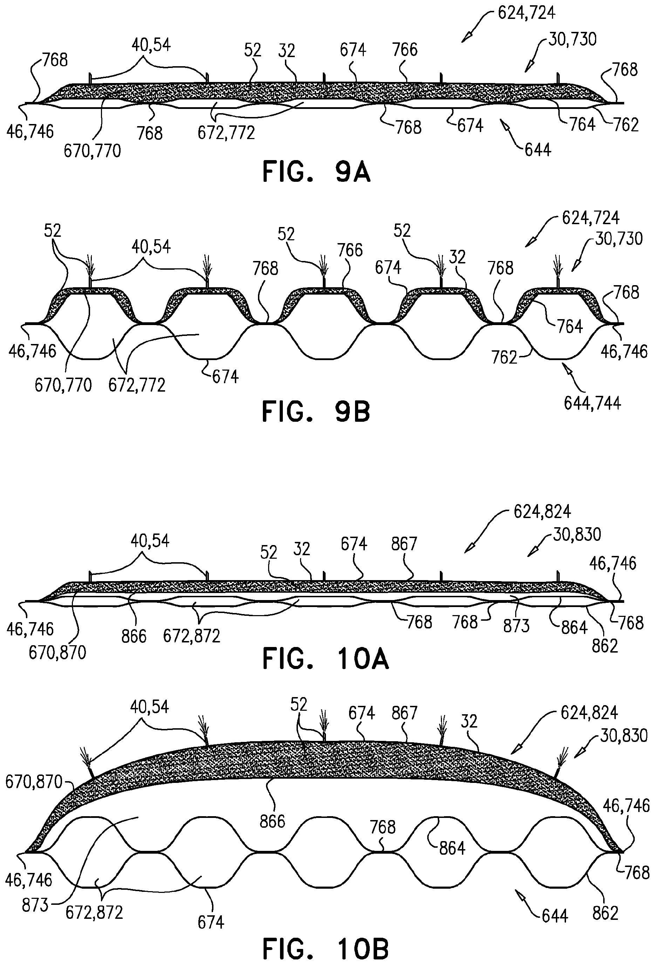

Optionally, patch 30 comprises a plurality of layers, which are fixed together (for example, fused (e.g., welded) or glued together) at one or more fixation locations 768, such as shown, for example, in Figs. 9A-B and 10A-B. All of the layers described herein may comprise one or more pieces of material, and multiple layers may optionally be formed from a single folded piece of material. The layers described herein comprise a broad, thin, pliable material, e.g., a membrane.

Reference is made to Figs. 7A-13D, which are schematic illustrations of several configurations of a medication-delivery device 624.

Medication-delivery device 624 may implement any of the features of medication-delivery device 24, described hereinabove with reference to Figs. 1A-6F. Medication-delivery device is a component of ingestible pill 20 (which comprises enteric coating 22), described hereinabove with reference to Figs. 1A- 6F.

Figs. 7A-C show medication-delivery device 624 in an expanded shape 644 after enteric coating 22 dissolves. Fig. 7B shows medication-delivery device 624 before expansion of one or more expansible chambers 672, as described below. Figs. 7A and 7C show medication-delivery device 624 upon expansion of the one or more expansible chambers 672, as described below.

In the configurations described with reference to Figs. 7A-13D, medication-delivery device 624 is pliable, i.e., easily bent and flexible, as opposed to stiff or rigid. Medication-delivery device 624 is disposed within enteric coating 22, having a compressed shape enabled by the pliability (similar to the configuration shown in Fig. 1A). The pliability also typically helps medication-delivery device 624 conform with intestinal wall 48 when in expanded shape 644.

Medication-delivery device 624 is shaped so as to define one or more medication chambers 670 and one or more expansible chambers 672. Medication-delivery device 624 comprises:
- one or more outer surfaces 674, all of which are pliable;
- hollow medication-delivery needles 54, which are coupled to at least one of the one or more outer surfaces 674; and
- medication 52, which is contained within the one or more medication chambers 670.

Medication-delivery device 624 is configured to assume, after enteric coating 22 dissolves, expanded shape 644 in which hollow medication-delivery needles 54 (a) are in fluid communication with the one or more medication chambers 670 and (b) extend away from medication-delivery device 624. Medication-delivery device 624 is configured such that expansion of the one or more expansible chambers 672 forces medication 52 from the one or more medication chambers 670 and out of medication-delivery device 624 through hollow medication-delivery needles 54. For example, medication 52 may comprise a liquid, or a solid that dissolves upon exposure to bodily fluids.

For some applications, the one or more expansible chambers 672 comprise one or more inflatable chambers. For some applications, the one or more inflatable chambers contain a substance that produces gas upon contact with liquid, such as described hereinbelow with reference to Figs. 9A-B.

For some applications, at least one of the one or more outer surfaces 674 comprises biocellulose (also known in the art as microbial cellulose and bacterial cellulose), which is liquid-permeable and substantially not gas-permeable.

Reference is made to Figs. 7A-8B. For some applications, medication-delivery device 624 is shaped as a circular or elliptical torus. The toroidal shape provides the above-mentioned structure.

Reference is made to Figs. 9A-13D. For some applications, medication-delivery device 624 is shaped as a patch 30.

Reference is made to Figs. 9A-B, which are schematic illustrations of a medication-delivery device 724.

Fig. 9A shows medication-delivery device 724 after enteric coating 22 has dissolved. Fig. 9B shows medication-delivery device 724 after expansion of expansible chambers 672, 772, as described hereinbelow. Medication-delivery device 724 is one implementation of medication-delivery device 624, described hereinabove, and may implement any of the features thereof. In addition, medication-delivery device 724 may implement any of the features of the medication-delivery devices described herein with reference to Figs. 1A-B, 2A-F, 3A-B, 4A-F, 5A-B, 6A-F, 11A-B, 12A-C, and/or 13A-D.

Medication-delivery device 724 is a component of an ingestible pill comprising enteric coating 22. Medication-delivery device 724 comprises a patch 730, which has a compressed shape when disposed within enteric coating 22, and which is shaped so as to define an outer perimeter 46, 746, and which comprises at least a first layer 762, a second layer 764, and a third layer 766, which are arranged so as to define:
- one or more medication chambers 670, 770 between second layer 764 and third layer 766, and
- one or more expansible chambers 672, 772 between first layer 762 and second layer 764.

First layer 762 and second layer 764 are fixed together at fixation locations 768, for example, fused (e.g., welded) or glued together. Some of fixation locations 768 are located at or near outer perimeter 760 and some of fixation locations 768 are located at least 2 mm from outer perimeter 760. Fixation locations 768 generally at least partially divide an interior of the one or more expansible chambers 672, 772 into sub-chambers, which help maintain patch 730 fairly flat even upon inflation.

Medication-delivery device 724 further comprises medication 52, which is contained within the one or more medication chambers 670, 770.

Patch 730 is configured to assume, after enteric coating 22 dissolves, an expanded shape 644, 744, such as shown in Fig. 9B.

Third layer 766 is permeable to medication 52, such that expansion of the one or more expansible chambers 672, 772 forces medication 52 from the one or more medication chambers 670, 770, through third layer 766, and out of medication-delivery device 724. Third layer 766 thus serves as upper surface 32 of patch 730 that contact intestinal wall 48, as described hereinabove.

For some applications, the one or more expansible chambers 672, 772 comprise one or more inflatable chambers. Typically, the one or more inflatable chambers contain a substance that produces gas upon contact with liquid. For example, the substance may comprise sodium bicarbonate and/or citric acid (e.g., 60-70% sodium bicarbonate and 30-40% citric acid). For some applications, first layer 762 is liquid-permeable and substantially not gas-permeable, in order to allow bodily fluids to pass into the one or more expansible chambers 672, 772 and contact the substance that produces the gas. For example, first layer 762 may comprise biocellulose. Optionally, the entire patch 730, except the upper surface that faces the intestinal wall, comprises biocellulose.

For other applications, the one or more expansible chambers 672, 772 contain a substance that expands upon contact with liquid. For example, the substance may comprise a polymer, such as a hydrogel.

For some applications, patch 730 can inscribe a circle having a diameter of at least 2 cm, less than 10 cm (e.g., less than 7 cm, such as less than 5 cm), and/or between 2 and 10 cm, e.g., between 2 and 7 cm, such as between 2 and 5 cm), when patch 30 assumes expanded shape 644, 744. Typically, patch 730 is configured such that expanded shape 644, 744 is generally flat when medication-delivery device 724 is unconstrained and becomes curved by intestinal wall 48.

For some applications, medication-delivery device 724 comprises a plurality of hollow medication-delivery needles 40, 54, which are coupled to third layer 766 in fluid communication with the one or more medication chambers 670 when medication-delivery device 724 is in expanded shape 644, 744, and extend away from patch 730 when patch 730 assumes expanded shape 644, 744. Typically, the ingestible pill is configured such that when patch 730 assumes expanded shape 644, 744 upon dissolving of enteric coating 22 in small intestine 26, third layer 766 of patch 30 contacts intestinal wall 48, thereby bringing hollow medication-delivery needles 54 into contact with intestinal wall 48.

For other applications, third layer 766 is shaped so as to define one or more pores that provide the permeability.

Reference is made to Figs. 10A-B, which are schematic illustrations of a medication-delivery device 824.

Fig. 10A shows medication-delivery device 824 after enteric coating 22 has dissolved. Fig. 10B shows medication-delivery device 824 after expansion of first expansible chambers 873 and second expansible chambers 672, 872, as described hereinbelow. Medication-delivery device 824 is one implementation of medication-delivery device 624, described hereinabove, and may implement any of the features thereof. In addition, medication-delivery device 824 may implement any of the features of the medication-delivery devices described herein with reference to Figs. 1A-B, 2A-F, 3A-B, 4A-F, 5A-B, 6A-F, 9A-B, 11A-B, 12A-C, and/or 13A-D.

Other than as described below, medication-delivery device 824 is identical to medication-delivery device 724, described hereinabove with reference to Figs. 9A-B.

A patch 830 of medication-delivery device 824 comprises at least a first layer 862, a second layer 864, a third layer 866, and a fourth layer 867, which are arranged so as to define:
- one or more medication chambers 670, 870 between third layer 866 and fourth layer 867,
- one or more first expansible chambers 873 between second layer 864 and third layer 866, and
- one or more second expansible chambers 672, 872 between first layer 862 and second layer 864.

First layer 862 and second layer 864 are fixed together at fixation locations 768. Some of the fixation locations 868 are located at or near outer perimeter 46, 746 and some of fixation locations 768 are located at least 2 mm from outer perimeter 46, 746.

Fourth layer 867 is permeable to medication 52, such that expansion of the one or more second expansible chambers 672, 872 forces medication 52 from the one or more medication chambers 670, 870, through fourth layer 867, and out of medication-delivery device 824. Fourth layer 867 thus serves as upper surface 32 of patch 830 that contact intestinal wall 48, as described hereinabove.

For some applications, medication-delivery device 824 is configured such that upon contact with liquid, the one or more second expansible chambers 672, 872 begin to expand before the one or more first expansible chambers 873 begin to expand. For example, the liquid may have separate access to the one or more second expansible chambers 672, 872 and the one or more first expansible chambers 873, and a physical property of the one of or more of the layers is configured to control timing. For some applications, the one or more first expansible chambers 873 comprise one or more first inflatable chambers, and second layer 864 is gas-permeable, such that upon inflation of the one or more second inflatable chambers, gas passes from the one or more second inflatable chambers through the gas-permeable second layer 864 to the one or more first inflatable chambers, such that the one or more second inflatable chambers begin to inflate before the one or more first inflatable chambers begin to inflate, which in turn force the medication 52 through fourth layer 867. For some applications, second layer 864 is shaped so as define a plurality of pores (i.e., small holes) that provides the gas-permeability. Alternatively or additionally, for some applications, second layer 864 is configured to tear upon inflation of the one or more second inflatable chambers, thereby providing the gas-permeability.

For some applications, first layer 862 is liquid-permeable and substantially not gas-permeable, such as described above with reference to Figs. 9A-B. For example, first layer 862 may comprise biocellulose.

For some applications, second layer 864 is liquid-permeable and substantially not gas-permeable. For some applications, first layer 862 comprises biocellulose.

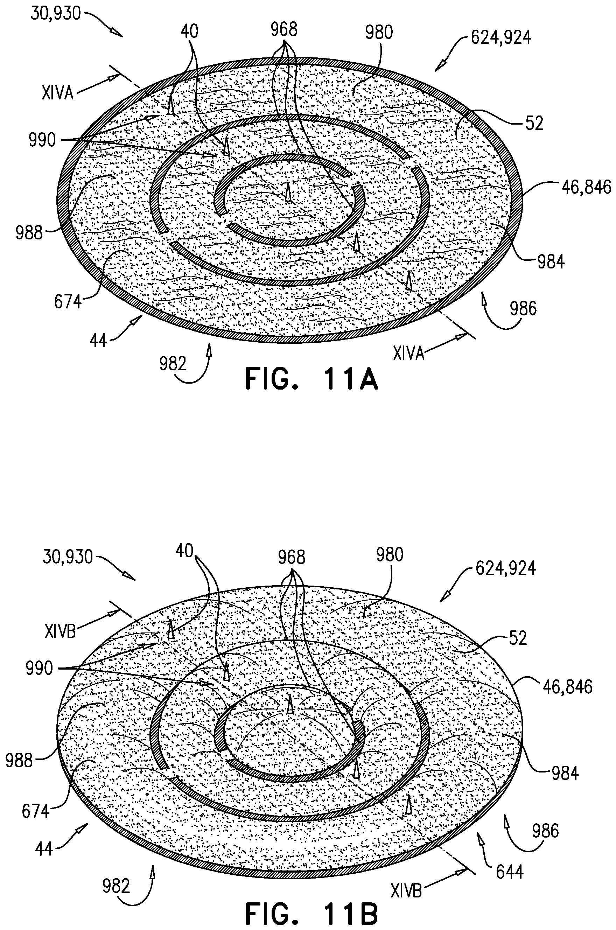

Reference is again made to Figs. 9A-B and 10A-B, and is additionally made to Figs. 11A-B, which are schematic illustrations of a medication-delivery device 924.

Medication-delivery device 924 is a component of an ingestible pill comprising enteric coating 22. Medication-delivery device 924 is one implementation of medication-delivery device 624, described hereinabove, and may implement any of the features thereof. In addition, medication-delivery device 924 may implement any of the features of the medication-delivery devices described herein with reference to Figs. 1A-B, 2A-F, 3A-B, 4A-F, 5A-B, 6A-F, 10A-B, 12A-C, and/or 13A-D.

Fig. 11A shows medication-delivery device 924 after enteric coating 22 has dissolved. Fig. 11B shows medication-delivery device 924 after expansion of expansible chambers 988 thereof. For example, medication-delivery device 924 may be shaped so as to define expansible chambers 772, such as described hereinabove with reference to Figs. 9A-B, or first expansible chambers 873 and second expansible chambers 872, such as described hereinabove with reference to Figs. 10A-B. The expansible chambers may implement any of the features of the expansible chambers described herein.

Medication-delivery device 924 comprises (a) a patch 930, which has a compressed shape when disposed within enteric coating 22, and which is shaped so as to define an outer perimeter 46, 846, and (b) needles 40.

Patch 930 has an upper surface 980 and a lower surface 982 that face in generally opposite directions. Patch 930 (iv) comprises at least a first layer 984 and a second layer 986, which are arranged so as to define one or more expansible chambers 988 between first layer 984 and second layer 986. First layer 984 and second layer 986 are fixed together at fixation locations 968. Typically, some of fixation locations 968 are located at or near outer perimeter 46, 846 and some of fixation locations 968 are located at least 2 mm from outer perimeter 46, 846. Needles 40 are coupled to upper surface 980 at respective needle locations 990.

Patch 930 is configured such that, if patch 930 were laid generally flat on a flat horizontal surface, at least 80%, e.g., at least 90%, such as all, of needle locations 990 would be horizontally offset from fixation locations 968. As used in the present application, including in the claims, "horizontally" means in a direction along the plane defined by the flat horizontal surface. It is noted that flat horizontal surface is not an element of the invention, but instead is a geometric shape used to define certain structural features of the invention. The invention does not require actually placing the patch laid flat on the flat horizontal surface.

For some applications, patch 930 is configured such that, if patch 930 were laid generally flat on the flat horizontal surface, needle locations 990 would be located on average at least 1 mm (e.g., at least 2 mm, such as at least 3 mm, e.g., at least 5 mm) horizontally from respective nearest fixation locations 968. For some applications, patch 930 is configured such that, if patch 930 were laid generally flat on the flat horizontal surface, each of needle locations 990 would be located at least 1 mm (e.g., at least 2 mm, such as at least 3 mm, e.g., at least 5 mm) horizontally from a nearest one of fixation locations 968.

For some applications, patch 930 is configured such that, if patch 930 were laid generally flat on the flat horizontal surface, each of needle locations 990 would be located approximately horizontally centered between a nearest two of fixation locations 968.

The arrangements of needle locations 990 with respect to fixation locations 968 described above result in needles 40 being generally located at or near local peaks of upper surface 980 between valleys defined by fixation locations 968. These relatively high locations may help maximize the puncturing of needles 40 through villi of intestinal wall 48 with as short as possible needles, and with minimal risk of blocking the needles with the expandable chamber before all of the drug is expelled (i.e., with minimum residual drug).

Needles 40 may have any of the features described herein. For example, the needles may comprise a solid medication, or may be configured to deliver a medication contained in medication-delivery device 924, such as in one or more medication chambers, such as described herein.

For some applications, patch 930 is disposed within enteric coating 22, folded so as to define one or more creases (e.g., a plurality of creases), which define respective inner and outer crease sides, and wherein at least 50% of the needles are coupled to upper surface 980 of patch 930 along the inner crease sides, such as described hereinabove with reference to Figs. 2A-F, 3A-B, 4A-F, 5A-B, and 6A-F.

For some applications, patch 930 can inscribe a circle having a diameter of at least 2 cm, less than 10 cm (e.g., less than 7 cm, such as less than 5 cm), and/or between 2 and 10 cm, e.g., between 2 and 7 cm, such as between 2 and 5 cm, when patch 930 assumes the expanded shape and is unconstrained.

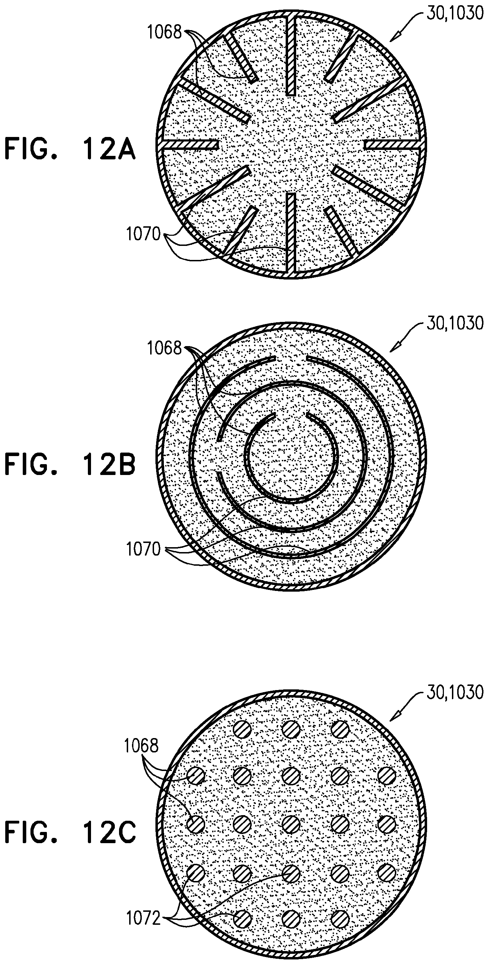

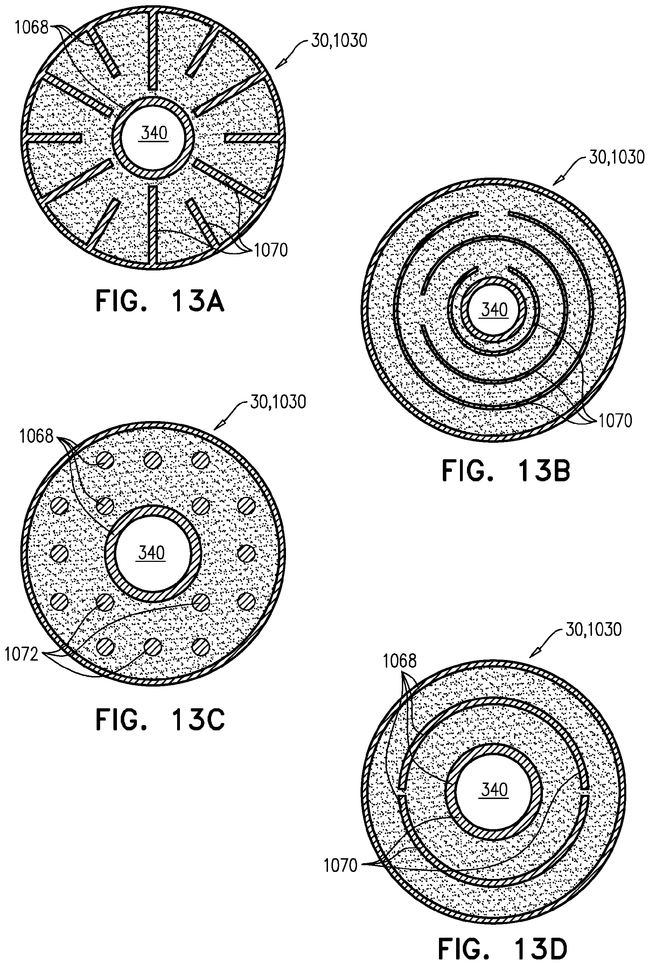

Reference is now made to Figs. 12A-C and 13A-D, which are schematic illustrations of configurations of a patch 30, 1030.

Any of the patches described herein that have fixation locations may implement any of these configurations. Fixation locations 1068 of patch 30, 1030 are between two or more layers of patch 30, 1030, as described hereinabove.

For some applications, at least some of fixation locations 1068 are arranged in a plurality of segments 1070. For some applications, at least some of segments 1070 are curved when patch 30 assumes expanded shape 44. For some of these applications, at least some of the curved segments 1070 are arranged equidistantly around a center point of patch 30 when patch 30 assumes expanded shape 44. For some applications, at least some of segments 1070 are straight when patch 30 assumes expanded shape 44. For some of these applications, at least some of straight segments are arranged radially when patch 30 assumes expanded shape 44, and may not reach a center point of the patch.

Alternatively or additionally, at least some of fixation locations 768 are arranged in a plurality of points 1072. As used in the present application, including the claims, "points" are small point-like areas rather than literal zero-dimensional points.

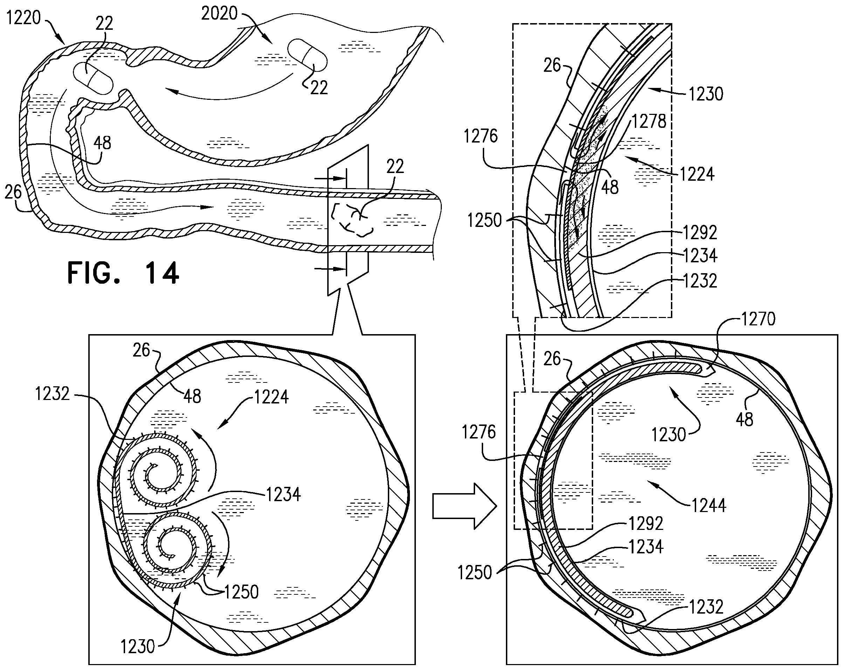

Reference is now made to Fig. 14, which is a schematic illustration of an ingestible pill 1220, for ingestion by a subject, in accordance with an application of the present invention. Ingestible pill 1220 comprises enteric coating 22, a medication 52, and a medication-delivery device 1224, which is configured to deliver medication 52.

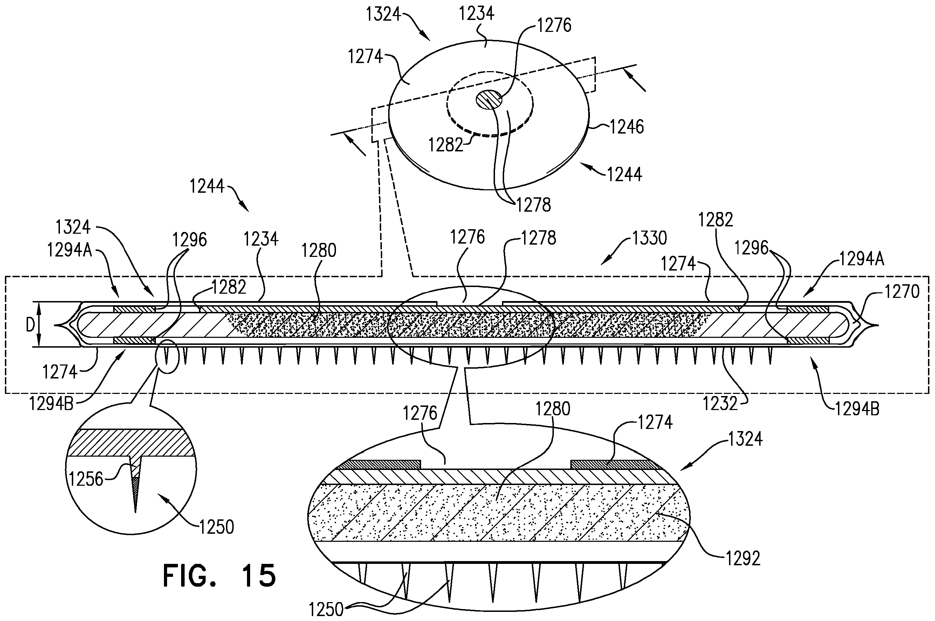

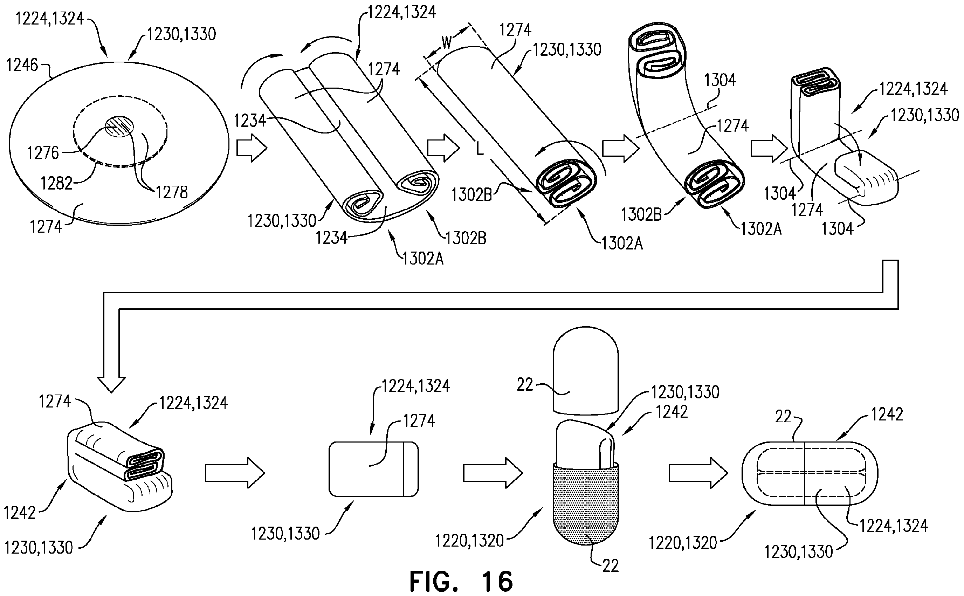

Medication-delivery device 1224 comprises a patch 1230 having an upper (intestinal-wall-contact) surface 1232 and a lower (intestinal-lumen-facing) surface 1234, which face in generally opposite directions. Patch 1230 has a compressed shape 1242 when disposed within enteric coating 22, as shown in Fig. 16, and is configured to assume, after enteric coating 22 dissolves, an expanded shape 1244, in which patch 1230 has an outer perimeter 1246 (labeled in medication-delivery device 1324, shown in Fig. 15).

Patch 1230 comprises a chamber 1270 defined by:
- a substantially water-impermeable and substantially gas-impermeable, elastic layer 1274 shaped so as to define at least one window 1276 (e.g., exactly one window 1276, or more than one window 1276) therethrough; for example, elastic layer 1274 may comprise a polymer such as a thermoplastic elastomer (TPE), which may, for example, comprise thermoplastic polyurethane (TPU); polyurethane (PU); or polyethylene (PE), and
- a liquid-permeable and substantially gas-impermeable wetting layer 1278, which entirely covers the at least one window 1276 and is sealed to elastic layer 1274 around the at least one window 1276; for example, wetting layer 1278 may comprise cellulose or biocellulose.

For some applications, as shown in Figs. 14 and 15, elastic layer 1274 is shaped so as to define upper (intestinal-wall-contact) surface 1232 and lower (intestinal-lumen-facing) surface 1234 (except at the location(s) of the at least one window 1276, at which wetting layer 1278 defines the surface. Alternatively or additionally, wetting layer 1278 partially or entirely defines upper (intestinal-wall-contact) surface 1232 and/or lower (intestinal-lumen-facing) surface 1234; this configuration may increase the contact surface between wetting layer 1278 and bodily fluids, thereby increasing the efficiency of wetting.

Patch 1230 further comprises a gas-generating substance 1280, disposed within chamber 1270, that produces gas upon contact with liquid in the intestine. Wetting layer 1278 allows bodily fluids to pass into chamber 1270 and contact gas-generating substance 1280, upon dissolving of enteric coating 22 in small intestine 26. The generation of the gas within chamber 1270 promotes transition of patch 1230 from compressed shape 1242 to expanded shape 1244. For configurations in which patch is rolled and/or folded within enteric coating 22, such as described, for example, hereinbelow with reference to Fig. 16, generation of the gas within chamber 1270 may promote unrolling/unfolding of patch 1230 and/or providing structure to patch 1230 that may push upper (intestinal-wall-contact) surface 1232 against intestinal wall 48. Alternatively or additionally, for configurations in which medication-delivery device 1224 comprises medication-needles 1250 or hollow medication-delivery needles (as described hereinbelow with reference to Figs. 14-18D), generation of the gas within chamber 1270 may push the needles against and/or into tissue of intestinal wall 48.

Typically, gas-generating substance 1280 is disposed in a vicinity of the at least one window 1276. For some applications, gas-generating substance 1280 comprises a powder. For some applications, gas-generating substance 1280 comprises sodium bicarbonate, citric acid, and/or a mixture of bicarbonate and citric acid (e.g., 60-70% sodium bicarbonate and 30-40% citric acid).

As described above, the layers described herein comprise a broad, thin, pliable material, e.g., a membrane.

As used in the present application, including in the claims, "substantially water-impermeable" and "substantially gas-impermeable" mean designed to prevent passage of water and gas, respectively, therethrough, it being understood that few materials are entirely water-impermeable or gas-impermeable.

Although window 1276 is shown as circular in the figures, this is by way of example only, and window 1276 may have any other shape, such as square, rectangular, polygonal, or elliptical. For some applications, the at least one window 1276 (e.g., exactly one window 1276, or more than one window 1276) has an aggregate area of at least 7 mm^2, no more than 55 mm^2, and/or between 7 and 55 mm^2, such as between 25 and 50 mm^2.

Elastic layer 1274 may comprise exactly one piece of material or a plurality of pieces of material (e.g., two pieces) that are sealed together to collectively define upper (intestinal-wall-contact) surface 1232 and lower (intestinal-lumen-facing) surface 1234.

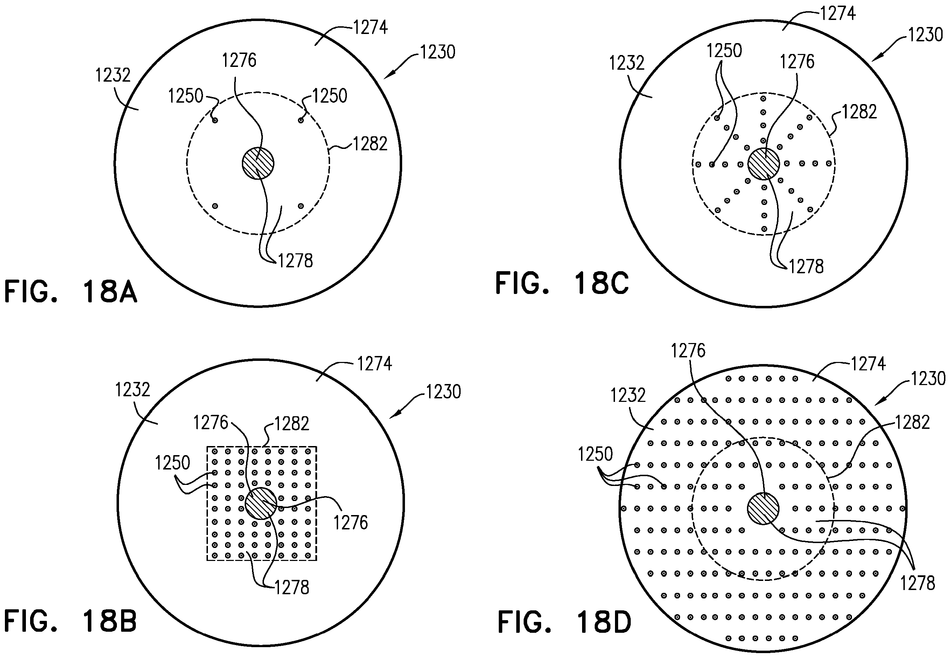

Wetting layer 1278 typically, although not necessarily, has a surface area greater than the area of window 1276, such that wetting layer 1278 overlaps with elastic layer 1274 in a region surrounding window 1276, in order to allow for good sealing of wetting layer 1278 to elastic layer 1274 around window 1276. An outer perimeter 1282 of wetting layer 1278 is shown by way of illustration and not limitation in the Figs. 15, 16, and 18A-D. For example, wetting layer 1278 may be sealed to elastic layer 1274 by fusing (e.g., welding) or gluing. Although wetting layer 1278 is shown as circular in most of the figures, this is by way of example only, and wetting layer 1278 may have any other shape, such as square (as shown in Fig. 18C), rectangular, polygonal, or elliptical. For some applications, wetting layer 1278 is sealed to an internal surface of elastic layer 1274 around the at least one window 1276. Alternatively, wetting layer 1278 is sealed to an external surface of elastic layer 1274 around the at least one window 1276 (optionally, wetting layer 1278 entirely encapsulates patch 1230).

Typically, patch 1230 is arranged within enteric coating 22 in compressed shape 1242 (e.g., rolled, folded, or rolled and folded), such that when patch 1230 assumes expanded shape 1244 after enteric coating 22 dissolves in small intestine 26, upper surface 1232 of patch 1230 contacts small intestinal wall 48, as shown in Fig. 14. For some applications, medication-delivery device 1224 is configured to release medication 52 such that at least 60% (e.g., at least 80%, such as at least 95%) of the released medication 52 is released at upper surface 1232 of patch 1230.

Reference is still made to Fig. 14, and is additionally made to Fig. 15, which is a schematic illustration of a medication-delivery device 1324 of an ingestible pill 1320, for ingestion by a subject, in accordance with an application of the present invention. Ingestible pill 1320 is shown in Fig. 16. Other than as described herein, ingestible pill 1320 and medication-delivery device 1324 are identical to ingestible pill 1220 and medication-delivery device 1224, respectively, and may implement any of the features of ingestible pill 1220 and medication-delivery device 1224, *mutatis mutandis.* Medication-delivery device 1324 comprises a patch 1330, which is identical to patch 1230, except as described below.

In medication-delivery device 1224, shown in Fig. 14, elastic layer 1274 is shaped so as to define the at least one window 1276 on an upper side of chamber 1270, such that the at least one window faces small intestinal wall 48 upon deployment. By contrast, in medication-delivery device 1324, shown in Fig. 15, elastic layer 1274 is shaped so as to define the at least one window 1276 on a lower side of chamber 1270, such that the at least one window faces away from small intestinal wall 48 upon deployment. In experiments conducted by the inventors, the inventors found that this latter orientation of the at least one window results in quicker flow of bodily fluid through the at least one window than if the at least one window faced the intestinal wall. The inventors believe that the slower flow observed when the at least one window faces the intestinal wall was caused by enteric coating 22 covering wetting layer 1278 upon transition of patch 1230/1330 to expanded shape 1244. In addition, disposing the at least one window 1276 on the lower side of chamber 1270 provides more surface area for medication 52 (e.g., medication-needles 1250) on upper surface 1232, because it is generally more difficult to dispose the medication or medication-needles on wetting layer 1278 over the at least one window. However, medication-needles 1250 may optionally be attached to wetting layer 1278, such as in configurations in which the at least one window 1276 is through upper surface 1232.

For some applications, medication-delivery device 1224/1324 further comprises a porous solid foam 1292 disposed within chamber 1270; typically, porous solid foam 1292 is shaped so as to define open pores, which allow fluid flow between pores, as is known in the foam art. Porous solid foam 1292 provides structure to medication-delivery device 1224/1324 to help maintain the device in a desired shape. For example, porous solid foam 1292 may comprise a polymer such as poly(vinyl alcohol) (PVA), polyurethane (PU), or polyethylene (PE), or silicone. As used in the present application, including the claims, the word "solid" in "solid foam" refers to the state of matter, rather than implying any level of rigidity of the foam.

For some applications, gas-generating substance 1280 is at least partially disposed in porous solid foam 1292 (i.e., within the pores of porous solid form 1292), and/or at least partially disposed between porous solid foam 1292 and wetting layer 1278.

For some applications, porous solid foam 1292 is fixed to one or more portions of an internal surface of elastic layer 1274 so as to inhibit expansion of chamber 1270 upon production of the gas by gas-generating substance 1280. This inhibition of expansion prevents chamber 1270 from becoming too spherical, thereby maintaining chamber 1270 shaped generally as a patch and/or swollen shape, e.g., swollen disc shape, when patch 1230/1330 assumes expanded shape 1244 and is unconstrained.. To this end, for some applications porous solid foam 1292 is fixed to (e.g., by an adhesive 1296) at least first and second portions 1294A and 1294B of the internal surface of elastic layer 1274 that respectively define upper and lower surfaces 1232 and 1234 of patch 1230/1330.

For some applications, porous solid foam 1292 is shaped as a disc. For example, the disc may have a diameter of at least 2 cm, no more than 10 cm, and/or between 2 and 10 cm.

For some applications, porous solid foam 1292 has a volume, when dry, of at least 0.5 cm^3, no more than 16 cm^3, and/or between 0.5 and 16 cm^3.

For some applications, porous solid foam 1292 has an average thickness of at least 1 mm, no more than 2 mm, and/or between 1 and 2 mm.

In some applications of the present invention, porous solid foam 1292 is integral with at least a portion of elastic layer 1274 (e.g., at least one piece of material of elastic layer 1274 for applications in which elastic layer 1274 comprises a plurality of pieces of material that are sealed together, as described hereinbelow). In other words, porous solid foam 1292 and the at least a portion of elastic layer 1274 are fabricated (e.g., molded) from a single piece of material. For example, the portion of the integral piece of material that defines porous solid foam 1292 may be shaped so as to define open pores, as described above, and the portion of the integral piece of material that defines the at least a portion of elastic layer 1274 may be shaped so as to define closed pores or no pores. For example, the integral material may comprise a polymer such as polyurethane (PU) or polyethylene (PE).

For some applications, medication 52 is solid and is shaped as at least respective portions of a plurality of medication-needles 1250 (for example, the solid medication may be integrated in a glucose structure). Typically, patch 1230/1330 is arranged within enteric coating 22 in compressed shape 1242, such that when the patch assumes expanded shape 1244 after enteric coating 22 dissolves in small intestine 26, upper (intestinal-wall-contact) surface 1232 of the patch contacts intestinal wall 48, thereby bringing medication-needles 1250 into contact with the intestinal wall.

Optionally, medication-needles 1250 further comprise respective non-medication support structures 1256. For example, non-medication support structures 1256 may serve as bases for medication-needles 1250, as shown in Fig. 15. For example, non-medication support structures 1256 may comprise a polymer, e.g., PVA or PU.

For some applications, at least 60% (e.g., at least 80%, such as at least 95%, e.g., 100%) of medication-needles 1250 are coupled to upper surface 1232 of patch 1230/1330.

For some applications, ingestible pill 1220/1320 comprises at least 4, no more than 150, and/or between 4 and 150 medication-needles 1250.

For some applications, each of medication-needles 1250 has a length of at least 20 microns (e.g., at least 50 microns), no more than 600 microns (e.g., no more than 300 microns, such as no more than 250 microns), and/or between 20 and 600 microns, e.g., between 20 and 600 microns, e.g., between 20 and 300 microns, such as between 50 and 250 microns. For some applications, an average density of all medication-needles 1250 on the surface to which medication-needles 1250 are attached is between 2 and 6 needles/mm2, such as 4 needles/mm2. Alternatively or additionally, for some applications, each of medication-needles 1250 is located within 1 mm of at least one adjacent medication-needle 1250.

For some applications, patch 1230/1330 can inscribe a circle having a diameter of at least 2 cm, less than 10 cm (e.g., less than 7 cm, such as less than 6 cm, e.g., less than 5 cm), and/or between 2 and 10 cm, e.g., between 2 and 7 cm, such as between 2 and 6, e.g., between 2 and 6 cm, when patch 1230/1330 assumes expanded shape 1244 and is unconstrained.

For some applications, a greatest distance D between upper and lower surfaces 1232 and 1234 of patch 1230/1330 is less than 6 mm, such as less than 4 mm, e.g., between 2 and 4 mm, such as between 3 and 4 mm, when patch 1230/1330 assumes expanded shape 1244 and is unconstrained (labeled in Fig. 15).

For some applications, a ratio between greatest distance D and a greatest dimension of patch 1230/1330 perpendicular to greatest distance D is less than 10%, e.g., less than 6%, such as less than 5%, and, optionally at least 1%, such as at least 2%, when patch 1230/1330 assumes expanded shape 1244 and is unconstrained.

For some applications, upper and lower surfaces 1232 and 1234 of patch 1230/1330 are substantially planar and, optionally, substantially parallel to each other, when patch 1230/1330 assumes expanded shape 1244 and is unconstrained, such as shown in Fig. 15.

Reference is now made to Fig. 16, which is a schematic illustration of a method of rolling patch 1230/1330 of medication-delivery device 1224/1324, in accordance with an application of the present invention. Fig. 16 also shows the rolled form disposed within enteric coating 22 in compressed shape 1242. For clarity of illustration, Fig. 16 shows both patch 1230 and patch 1330 schematically without needles. Patch 1230/1330 may also be rolled, folded, or rolled and folded in other ways.

In the configuration illustrated in Fig. 16, upon rolling, patch 1230/1330 is disposed within enteric coating 22 in compressed shape 1242, in which two opposite perimeter portions 1302A and 1302B of outer perimeter 1246 are (a) rolled toward each other over lower surface 1234 of patch 1230/1330 (as shown) or (b) folded toward each other over lower surface 1234 of patch 1230/1330 (not shown), such that, in either case, the two opposite perimeter portions 1302A and 1302B touch lower surface 1234, Typically, but not necessarily, patch 1230/1330 is disposed within enteric coating 22 in compressed shape 1242, in which the two opposite perimeter portions 1302A and 1302B of outer perimeter 1246 are (a) rolled at least one full turn toward each other over lower surface 1234 of patch 1230/1330 (as shown) or (b) folded at least one full turn toward each other over lower surface 1234 of patch 1230/1330 (not shown). As a result, patch 1230/1330 is rolled or folded into two separate spirals in cross section. As used in the present application, including in the claims, "folding" the patch typically does not result in sharp creases, although it optionally may.

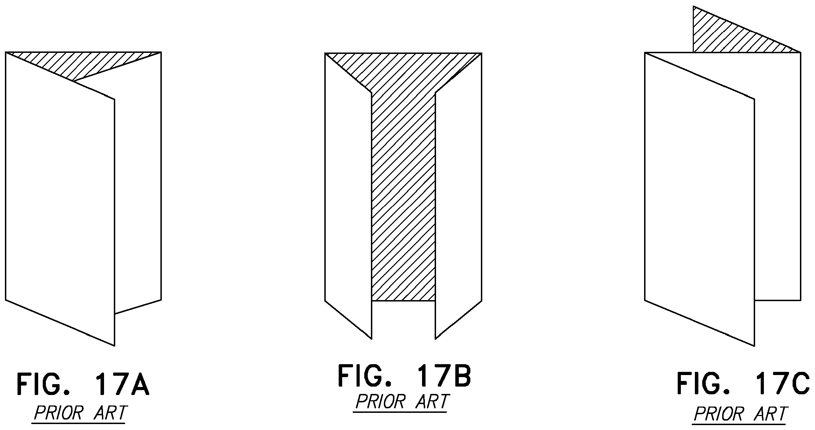

For some applications, patch 1230/1330 is disposed within enteric coating 22 in compressed shape 1242, in which:
- first, the two opposite perimeter portions 1302A and 1302B of outer perimeter 1246 are (a) rolled toward each other over lower surface 1234 of patch 1230/1330 (as shown) or (b) folded toward each other over lower surface 1234 of patch 1230/1330 (not shown), such that, in either case, the rolled or folded patch 1230/1330 has a length L greater than a width W thereof (e.g., at least 2 times, such as at least 3 times greater than width W), and
- then, the rolled or folded patch 1230/1330 is folded widthwise (i.e., folding about one or more lines 1304 that run in the direction of width W); optionally, the rolled or folded patch 1230/1330 is three-panel folded widthwise (as shown in Fig. 16), such as tri-folded (as shown in Fig. 16), three-panel gate folded (not shown in Fig. 16; see Fig. 17B, described below, or three-panel Z-folded (not shown in Fig. 16; see Fig. 17C, described below).

In experiments conducted by the inventors, the inventors have found that three-panel folding allows the folded patch 1230/1330 to readily unfold within a tube having a diameter of 25 mm (the approximate diameter of the human small intestine, which is typically between 25 and 30 mm), and within small intestines of pigs, because each of the folds is less than the diameter of the tube. By contrast, the inventors have found that two-panel folding, i.e., folding in half, prevents the folded patch 1230/1330 from unfolding within the 25-mm tube, because the wall of the tube blocks the unfolding. The inventors found that good unfolding and internal wall covering was achieved with a patch dimeter of between 1/2 and 2/3 of the perimeter of the tube/small intestine.

Reference is now made to Figs. 17A-C, which are schematic illustrations of three-panel folds known in the art of paper folding. These three known paper three-panel folds are shown in order to define the three-panel folds described immediately above. Fig. 17A shows a paper tri-fold (also known as a three-pane rolled fold). Fig. 17B shows a paper three-panel gate fold. Fig. 17C shows a three-panel Z-fold (also known a three-panel accordion fold). As used in the present application, "three-panel folded" includes these three three-panel folds, as well as other three-panel folds not illustrated.

For some applications, patch 1230/1330 is disposed within enteric coating 22 in compressed shape 1242, in which:
- first, patch 1230/1330 is rolled so as to form a single spiral in cross-section, such that the rolled patch 1230/1330 has a length greater than a width thereof (e.g., at least 2 times, such as at least 3 times greater than the width), and
- then, the rolled or folded patch 1230/1330 is folded widthwise (i.e., folding about one or more lines that run in the direction of the width); optionally, the rolled or folded patch 1230/1330 is three-panel folded widthwise, such as tri-folded (see Fig. 17A), three-panel gate folded (see Fig. 17B), described above, or three-panel Z-filed (see Fig. 17C, described above)).

Reference is now made to Figs. 18A-D, which are schematic illustrations of several configurations of patch 1230, in accordance with respective applications of the present invention. Figs. 18A-D show upper (intestinal-wall-contact) surface 1232 of patch 1330, with respective arrays of medication-needles 1250. For example, medication-needles 1250 may be distribute over only a portion of upper surface 1232 (such as a central portion), as shown in Figs. 18A-C, or over generally all of upper surface 1232, as shown in Fig. 18D.

Reference is made to Figs. 14-18D. For some applications, medication-delivery device 1224/1324 comprises hollow medication-delivery needles, which are coupled to (and typically protrude from) at least one of upper surface 1232 and lower surface 1234, and are configured to deliver medication 52. In these applications, patch 1230/1330 comprises one or more medication chambers, which contains medication 52, and the hollow medication-delivery needles in fluid communication with the one or more medication chambers. For example, the one or more medication chambers may be implemented using techniques described hereinabove regarding medication chambers 670, 770, and/or 870, *mutatis mutandis.* The hollow medication-delivery needles may optionally have any of the characteristics, including, but not limited to, locations, number, and dimensions, of medication-needles 1250.

The inventors conducted an experiment to test a medication-delivery device similar to medication-delivery device 1224/1324, described hereinabove with reference to Fig. 16. A stomach and three meters of the small intestine were harvested from a pig. The small intestine, together with the intestinal fluid, were transferred to an operation table. The experiment was performed under X-ray observation. The medication-delivery device, in a gelatin capsule, was inserted into the small intestine using an applicator. The patch unfolded and unrolled according to design, as shown schematically in Fig. 14.

In an embodiment, techniques and apparatus described in one or more of the following applications are combined with techniques and apparatus described herein:
- US Patent 8,287,902 to Gross;
- US Patent 9,492,396 to Gross; and/or
- US Patent Application 16/103,420, filed August 14, 2018.

## Claims

1. An ingestible pill comprising:
an enteric coating (22);
a medication; and
a medication-delivery device (1224) that comprises medication-needles (1250) or hollow medication-delivery needles, which is configured to deliver the medication and comprises a patch (1230) having upper and lower surfaces (1232, 1234) that face in generally opposite directions, and an outer perimeter (1246),
wherein the patch (1230) is disposed within the enteric coating (22) in a compressed shape (1242), in which two opposite perimeter portions of the outer perimeter (1246) are (a) rolled toward each other over the lower surface (1234) of the patch (1230) or (b) folded toward each other over the lower surface (1234) of the patch (1230), such that, in either case, the two opposite perimeter portions touch the lower surface (1234),
wherein the patch (1230) is configured to assume an expanded shape (1244) after the enteric coating dissolves.

2. The ingestible pill according to claim 1, wherein said patch (1230) comprises a gas-generating substance (1280) disposed within a chamber (1270) that produces gas upon contact with liquid in the intestine (26), generation of the gas within said chamber (1270) promoting transition of said patch (1230) from said compressed shape (1242) to said expanded shape (1244), and wherein generation of the gas within said chamber (1270) pushes said medication-needles (1250) or said hollow medication-delivery needles against and/or into tissue of the intestinal wall (48).

3. The ingestible pill according to claim 2, wherein said chamber (1270) comprises a substantially water-impermeable and substantially gas-impermeable, elastic layer (1274).

4. The ingestible pill according to claim 3, wherein said elastic layer (1274) is shaped so as to define at least one window (1276).

5. The ingestible pill according to claim 3 or 4, wherein said elastic layer (1274) comprise a polymer.

6. The ingestible pill according to any one of claims 3 to 5, wherein said elastic layer (1274) comprise a thermoplastic elastomer.

7. The ingestible pill according to any one of claims 4 to 6, wherein a liquid-permeable and substantially gas-impermeable wetting layer (1278) covers the at least one window (1276) and is sealed to said elastic layer (1274) around the at least one window.

8. The ingestible pill according to any one of claims 2 to 7, wherein said gas-generating substance (1280) comprises a powder.

9. The ingestible pill according to any one of claims 2 to 8, wherein said gas-generating substance (1280) comprises sodium bicarbonate.

10. The ingestible pill according to any one of claims 2 to 9, wherein said gas-generating substance (1280) comprises citric acid.

## Patentansprüche

1. Einnehmbare Pille, Folgendes umfassend:
eine magensaftresistente Beschichtung (22);
ein Medikament; und
eine Medikamentenabgabevorrichtung (1224), die Medikamentennadeln (1250) oder hohle Medikamentenabgabenadeln umfasst, die so konfiguriert ist, dass sie das Medikament abgibt, und ein Pflaster (1230) mit einer oberen und einer unteren Fläche (1232, 1234), die in allgemein entgegengesetzte Richtungen weisen, und einem Außenumfang (1246) umfasst,
wobei das Pflaster (1230) innerhalb der magensaftresistenten Beschichtung (22) in einer komprimierten Form (1242) angeordnet ist, in der zwei gegenüberliegende Umfangsabschnitte des Außenumfangs (1246) (a) über die untere Oberfläche (1234) des Pflasters (1230) aufeinander gerollt oder (b) über die untere Oberfläche (1234) des Pflasters (1230) aufeinander gefaltet sind, so dass in beiden Fällen die beiden gegenüberliegenden Umfangsabschnitte die untere Fläche (1234) berühren,
wobei das Pflaster (1230) so konfiguriert ist, dass es nach dem Auflösen der magensaftresistenten Beschichtung eine expandierte Form (1244) annimmt.

2. Einnehmbare Pille nach Anspruch 1, wobei das Pflaster (1230) eine gaserzeugende Substanz (1280) umfasst, die innerhalb einer Kammer (1270) angeordnet ist, die bei Kontakt mit Flüssigkeit im Darm (26) Gas erzeugt, wobei die Erzeugung des Gases innerhalb der Kammer (1270) den Übergang des Pflasters (1230) von der komprimierten Form (1242) in die expandierte Form (1244) fördert, und wobei die Erzeugung des Gases innerhalb der Kammer (1270) die Medikamentennadeln (1250) oder die hohlen Medikamentenabgabenadeln gegen und/oder in das Gewebe der Darmwand (48) drückt.

3. Einnehmbare Pille nach Anspruch 2, wobei die Kammer (1270) eine im Wesentlichen wasserundurchlässige und im Wesentlichen gasundurchlässige, elastische Schicht (1274) umfasst.

4. Einnehmbare Pille nach Anspruch 3, wobei die elastische Schicht (1274) so geformt ist, dass sie mindestens ein Fenster (1276) definiert.

5. Einnehmbare Pille nach Anspruch 3 oder 4, wobei die elastische Schicht (1274) ein Polymer umfasst.

6. Einnehmbare Pille nach einem der Ansprüche 3 bis 5, wobei die elastische Schicht (1274) ein thermoplastisches Elastomer umfasst.

7. Einnehmbare Pille nach einem der Ansprüche 4 bis 6, wobei eine flüssigkeitsdurchlässige und im Wesentlichen gasundurchlässige Benetzungsschicht (1278) das mindestens eine Fenster (1276) bedeckt und mit der elastischen Schicht (1274) um das mindestens eine Fenster herum versiegelt ist.

8. Einnehmbare Pille nach einem der Ansprüche 2 bis 7, wobei die gaserzeugende Substanz (1280) ein Pulver umfasst.

9. Einnehmbare Pille nach einem der Ansprüche 2 bis 8, wobei die gaserzeugende Substanz (1280) Natriumbikarbonat umfasst.

10. Einnehmbare Pille nach einem der Ansprüche 2 bis 9, wobei die gaserzeugende Substanz (1280) Zitronensäure umfasst.

## Revendications

1. Comprimé ingérable comprenant :
un enrobage entérique (22) ;
un médicament ; et
un dispositif d'administration de médicament (1224) qui comprend des aiguilles à médicament (1250) ou des aiguilles creuses d'administration de médicament, lequel est configuré pour administrer le médicament et comprend un timbre (1230) comportant des surfaces supérieure et inférieure (1232, 1234) qui se font face dans des directions généralement opposées, et un périmètre externe (1246) ;
dans lequel le timbre (1230) est disposé à l'intérieur de l'enrobage entérique (22) sous une forme comprimée (1242) dans laquelle deux parties périphériques opposées du périmètre externe (1246) sont (a) roulées en direction l'une de l'autre au-dessus de la surface inférieure (1234) du timbre (1230) ou (b) repliées en direction l'une de l'autre au-dessus de la surface inférieure (1234) du timbre (1230), de telle sorte que, quel que soit le cas, les deux parties périphériques opposées touchent la surface inférieure (1234) ;
dans lequel le timbre (1230) est configuré pour prendre une forme expansée (1244) après que l'enrobage entérique est dissous.

2. Comprimé ingérable selon la revendication 1, dans lequel ledit timbre (1230) comprend une substance gazogène (1280) disposée à l'intérieur d'une chambre (1270) qui produit du gaz suite à son contact avec du liquide dans l'intestin (26), la génération du gaz à l'intérieur de ladite chambre (1270) favorisant la transition dudit timbre (1230) de ladite forme comprimée (1242) à ladite forme expansée (1244), et dans lequel la génération du gaz à l'intérieur de ladite chambre (1270) pousse lesdites aiguilles à médicament (1250) ou lesdites aiguilles creuses d'administration de médicament contre et/ou à l'intérieur du tissu de la paroi intestinale (48).

3. Comprimé ingérable selon la revendication 2, dans lequel ladite chambre (1270) comprend une couche élastique sensiblement imperméable à l'eau et sensiblement imperméable au gaz (1274).

4. Comprimé ingérable selon la revendication 3, dans lequel ladite couche élastique (1274) est formée de manière à définir au moins une fenêtre (1276).

5. Comprimé ingérable selon la revendication 3 ou 4, dans lequel ladite couche élastique (1274) comprend un polymère.

6. Comprimé ingérable selon l'une quelconque des revendications 3 à 5, dans lequel ladite couche élastique (1274) comprend un élastomère thermoplastique.

7. Comprimé ingérable selon l'une quelconque des revendications 4 à 6, dans lequel une couche de mouillage perméable au liquide et sensiblement imperméable au gaz (1278) recouvre l'au moins une fenêtre (1276) et est scellée sur ladite couche élastique (1274) autour de l'au moins une fenêtre.

8. Comprimé ingérable selon l'une quelconque des revendications 2 à 7, dans lequel ladite substance gazogène (1280) comprend une poudre.

9. Comprimé ingérable selon l'une quelconque des revendications 2 à 8, dans lequel ladite substance gazogène (1280) comprend du bicarbonate de sodium.

10. Comprimé ingérable selon l'une quelconque des revendications 2 à 9, dans lequel ladite substance gazogène (1280) comprend de l'acide citrique.
